(19) Europäisches Patentamt
European Patent Office
Office européen des brevets

(11) **EP 3 271 738 B1**

(12) **EUROPEAN PATENT SPECIFICATION**

(45) Date of publication and mention
of the grant of the patent:
**08.05.2019 Bulletin 2019/19**

(21) Application number: **16710221.9**

(22) Date of filing: **17.03.2016**

(51) Int Cl.:
*G01R 33/56* *(2006.01)*     *G06T 7/00* *(2017.01)*
*A61B 5/055* *(2006.01)*     *G01R 33/561* *(2006.01)*

(86) International application number:
**PCT/EP2016/055806**

(87) International publication number:
**WO 2016/146745 (22.09.2016 Gazette 2016/38)**

(54) **COMPUTERIZED OPTICAL ANALYSIS METHODS OF MR (MAGNETIC RESONANCE) IMAGES FOR QUANTIFYING OR DETERMINING LIVER LESIONS**

RECHNERGESTÜTZTES OPTISCHES ANALYSEVERFAHREN VON MR (MAGNETRESONANZ)-BILDERN ZUR QUANTIFIZIERUNG ODER BESTIMMUNG VON LEBERLÄSIONEN

PROCÉDÉS D'ANALYSE OPTIQUE INFORMATISÉE D'IMAGES PAR RM (RÉSONANCE MAGNÉTIQUE) POUR LA QUANTIFICATION OU LA DÉTERMINATION DE LÉSIONS DU FOIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.03.2015 EP 15382118**

(43) Date of publication of application:
**24.01.2018 Bulletin 2018/04**

(73) Proprietors:
- **Servicio Andaluz de Salud**
  **41071 Sevilla (ES)**
- **Universidad De Sevilla**
  **41013 Sevilla (ES)**
- **Centro de Investigación Biomédica en Red (CIBER)**
  **28029 Madrid (ES)**

(72) Inventors:
- **ROMERO GÓMEZ, Manuel**
  **41071 Sevilla (ES)**

- **GÓMEZ GONZÁLEZ, Emilio**
  **41013 Sevilla (ES)**
- **GALLEGO DURÁN, Rocío**
  **28029 Madrid (ES)**
- **CERRO SALIDO, Pablo**
  **41013 Sevilla (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(56) References cited:
**KR-B1- 101 423 835**     **US-B2- 8 000 769**

- **CORBIN I.R. ET AL.: "Towards Noninvasive Biomarkers of Non-Alcoholic Steatohepatitis", INTERNATIONAL SOCIETY FOR MAGNETIC RESONANCE IN MEDICINE, 7 May 2005 (2005-05-07), page 337, XP040593021, Miami, Florida, USA**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

This invention relates to the field of hepatic diagnosis and more precisely to computerized optical analysis methods of MR (magnetic resonance) images for quantifying or determining liver lesions, especially due or related to liver impairment, liver steatosis, non-alcoholic fatty liver disease (NAFLD), or non-alcoholic steatohepatitis (NASH).

### Background of the invention

FLD (Fatty Liver Disease) describes a wide range of potentially reversible conditions involving the liver, wherein large vacuoles of triglyceride fat accumulate in hepatocytes via the process of steatosis (i.e. the abnormal retention of lipids within a cell). FLD is commonly associated with alcohol or metabolic syndromes (diabetes, hypertension, dyslipidemia, abetalipoproteinemia, glycogen storage diseases, Weber-Christian disease, Wolman disease, acute fatty liver of pregnancy, lipodystrophy). However, it can also be due to nutritional causes (malnutrition, total parenteral nutrition, severe weight loss, refeeding syndrome, jejuno-ileal bypass, gastric bypass, jejunal diverticulosis with bacterial overgrowth), as well as various drugs and toxins (amiodarone, methotrexate, diltiazem, highly active antiretroviral therapy, glucocorticoids, tamoxifen, environmental hepatotoxins) and other diseases such as inflammatory bowel disease or HIV.

Whether it is AFLD (Alcoholic Fatty Liver Disease) or NAFLD (Non-Alcoholic Fatty Liver Disease), FLD encompasses a morphological spectrum consisting from the mildest type "liver steatosis" (fatty liver), called NAFL, to the potentially more serious type "steatohepatitis", called NASH, which is associated with liver- damaging inflammation and, sometimes, the formation of fibrous tissue. In fact, steatohepatitis has the inherent propensity to progress towards the development of fibrosis then cirrhosis which can produce progressive, irreversible liver scarring or towards hepatocellular carcinoma (liver cancer).

Because these diseases can be potentially reversed if diagnosed early enough, or at least their consequences limited, it is crucial to be able to provide the medical field with tools that permitting such an early, rapid and precise diagnosis.

For a long time, the diagnosis of liver steatosis has usually been accomplished by measuring markers such as g-glutamyl- transpeptidase (GGT) and alanine aminotransferase (ALT) while at the same time performing a liver biopsy in order to confirm FLD and determine the grading and staging of the disease. Although biopsies can provide important information regarding the degree of liver damage, in particular the severity of necro-inflammatory activity, fibrosis and steatosis, the procedure also presents several limitations, such as sampling error, invasiveness, cost, pain for patients which in turn brings forth a certain reluctance to undergo such a procedure; and finally complications may arise from such procedure, which in some cases can even lead to mortality. Ultrasonography is also used to diagnose liver steatosis. However, this method is subjective as it is based on echo intensity (echogenicity) and special patterns of echoes (texture). As a result, it is not sensitive enough and often inaccurate in patients with advanced fibrosis. Finally, it is generally admitted that around half of all FLD cases are detected by usual blood tests and around half by ultrasonography, resulting in around one quarter of missed diagnosis when both are used.

In recent years, the use of non-invasive biomarkers has gained importance in the field of hepatic diagnosis. Indeed, several tests using non-invasive biomarkers have already been developed and proposed for the diagnosis of fibrosis (see for example WO 2005/116901). The test relates to a method of diagnosing the presence and/or severity of a hepatic pathology and/or of monitoring the effectiveness of a curative treatment against a hepatic pathology in an individual, by establishing at least one non-invasive diagnostic score, in particular a diagnostic score for portal and septal fibrosis and/or an estimate for amount of fibrosis (the area of fibrosis) and/or an estimate for the architecture of fibrosis (fractal dimension). In addition, Poynard et al. (WO 2006/082522) has demonstrated that a single or a panel of biomarkers can be used as an alternative to liver biopsy for the diagnosis of steatosis, whether induced by alcohol, viral hepatitis or NAFLD, the most common causes of steatosis. In particular, this document provides for a new panel of biomarkers known as a SteatoTest (ST) with predictive values for the diagnosis of steatosis due to alcohol, NAFLD and hepatitis C and B. Serum GGT and ALT were considered as the standard biochemical markers. However, according to the French National Agency for Health (HAS), updated in December 2008, and current international opinion, the performance of this kind of test may be insufficient, especially due to the reference based on a subjective grading of liver steatosis with a poor inter-observer reproducibility. In addition, this grading is a semi-quantitative variable which implies an imprecise and limited reflect of the original pattern.

The Applicant has now observed that computerized optical analysis methods of conventional non-contrast-enhanced MR (magnetic resonance) images of the liver enable the detection of steatohepatitis by NASH-MRI and significant fibrosis by Fibro-MRI in patients suffering from NAFLD. These methods circumvent the above-mentioned limitations and are more precise and reliable than the methods cited in the prior art, as shown by their high diagnostic performance. The methods of the invention considerably reduce the need of biopsies, as they catch more information than in the prior art about the lesions evaluated, they ensure reproducibility and performance, while attenuating causes of false results (generally, sources of false results are antagonized in a score including several markers provided that some precautions are included).

## Brief description of the invention

The present invention provides computerized optical analysis methods of MR (magnetic resonance) images of the liver, said methods enable the detection of steatohepatitis by NASH-MRI and significant fibrosis by Fibro-MRI in patients suffering from NAFLD. By using the methods of the present invention it is possible to forecast the rate of disease progression, to support therapeutic decision-making, and to monitor potential therapeutic effects.

In particular, a first aspect of the invention refers to an non-invasive method for quantifying the lesions of the liver of a subject suspected of suffering from a non-alcoholic fatty liver disease addressing as diagnostic target steatohepatitis. The steps of the method are set out in claim 1.

A second aspect of the invention refers to a non-invasive method for quantifying the lesions of the liver of a subject suspected of suffering from a non-alcoholic fatty liver disease addressing as diagnostic target fibrosis. The steps of the method are set out in claim 2.

In addition, it is also further noted that the method of the invention can be used in a method of treatment for treating those patients suffering form or having probabilities of suffering from any of the diagnostic targets. Such treatment therapy, in particular for NASH, can be selected from, but is not limited to, any of the following: OCA, elefibranor, aramchol, simtuzumab, IMM-124E, cernicriviroc, emricasan, GS-4997 + simtuzumab, Px-104, BMS-986036, SHP626, ZGN839, GR-MD-02, NDI-010976, GLP-01 semaglutide, NGM282, MSDC-0602, VK2809, MN-001, GS4998, RG-125, DUR-928, CER-209, solitromycin and PXS-4728A.

The invention also concerns a computerized optical analysis method in accordance with claim 3.

A further aspect of the invention refers to a computer program according to claim 4 for implementing the computerized optical analysis method of the precedent paragraph and a device comprising the computer program.

The present invention thus offers a clear solution to the problems cited above because it is focused on an in vitro method for identifying or screening human subjects at risk of suffering from steatohepatitis or fibrosis.

## Brief description of the figures

**Fig. 1.** Comparative analysis of diagnostic accuracy between NASHMRi (AUROC: 0.86;95%CI:0.76-0.96), CK-18 (AUROC:0.44;95%CI:0.29-0.60);p<0.0001.

**Fig. 2.** Comparative analysis of diagnostic accuracy between Fibro-MRI (AUROC: 0.95;95%CI:0.88-1.00) and Transient Elastography (AUROC:0.91;95%CI:0.81- 1.00);p=ns.

**Fig. 3.** Comparative analysis of diagnostic accuracy among FibroMRi (AUROC: 0.89;95%CI:0.79-0.98), Sydney Index (AUROC:0.64;95%CI:0.47-0.82) and NAFLD Fibrosis Score (AUROC: 0.61; 95%CI: 0.43-0.79); p<0.001.

## Description of the invention

- List of abbreviations:

**NAFLD:** Non-alcoholic fatty liver disease

**MRI:** Magnetic resonance images

**FGF21:** Fibroblast grown factor 21

**CK-18:** Cytokeratin 18

**NASH:** Non-alcoholic steatohepatitis

**NAS Score:** NAFLD Activity Score

**SAF:** Steatosis, Activity, Fibrosis score

**HOMA-IR:** Homeostatic Model Assessment of Insulin Resistance

**ALT:** Alanine aminotransferase

**AST:** Aspartate aminotransferase

**ECLIA:** Electrochemiluminescence immunoassay

**BMI:** Body mass index

**WL:** Window level

**WW:** Window width

**FoV:** Field of view

**ROC:** Receiver operating characteristics

**AUROC:** Area under receiver operating characteristic

**AUC:** Area under the curve

**PPV:** Positive predictive value

**NPV:** Negative predictive value

**MRS:** Magnetic resonance spectroscopy

**MRE:** Magnetic resonance elastography

## Definitions

In the context of the present invention E3: Harmonic mean is understood as the Harmonic mean $HH$ of a set of $nn$ numbers $\{xx1, xx2, \cdots xxnn\}$ as a specific kind of average. In this case, $\{xx1, 2, \cdots xxnn\}$ represents the whole set of pixel values of each image sample.

$$H = \frac{n}{\sum_{i=1}^{n} \frac{1}{x_i}}$$

In the context of the present invention E6: Mode means the Mode of a set of $nn$ numbers $\{xx1, 2, \cdots xxnn\}$, namely the mode is the value that occurs most often.

In the context of the present invention E22: Pearson Mode Skewness is the Pearson mode skewness, also called Pearson's first coefficient of skewness, is a way to figure out the skewness of a distribution. It tells how far the distribution departs from symmetry.

$$P = \frac{mean - mode}{\sigma}$$

In the context of the present invention E31: Column's mean in multi-oriented co-occurrence matrix is understood as follows. In image processing, co-occurrence matrices are used to analyze the texture of an image. A multi-oriented matrix computes the magnitude and orientation of the local gradient vector at each pixel position, so each pixel carries its gray value, its gradient magnitude and its gradient orientation.

In the context of the present invention E57: 2-2 Order Contrast is understood as the texture attribute of a given sample, computed as a local grey level variation in the grey level co-occurrence matrix. It can be thought of as a linear dependency of grey levels of neighboring pixels. If the neighboring pixels are very similar in their grey level values then the contrast in the image is very low. In case of texture, the grey level variations show the variation of texture itself. High contrast values are expected for heavy textures and low for smooth, soft textures. Specific details of this parameter are given in Texture Characterization based on Grey-Level Co-occurrence Matrix. Huertas, R. y Gebejes, A. 2013. Conference of Informatics and Management Sciences. 375-378.

In the context of the present invention E73: Averaged mean curvature is a value representing the mean of the curvatures mean at each pixel within the image sample. Curvature is a feature used to describe image surface.

In the context of the present invention E75: Maximum of primary curvatures is the maximum value for all the primary curvatures (primary curvature from every pixel of the sample) is used as a surface attribute descriptor.

In the context of the present invention the MRI protocols shown below can be followed in order to obtain the necessary information to carried-out the methods of the present invention. However, it is noted that the protocols shown herein below are especially suitable for acquiring MRIs using General Electric (Milwaukee, CT. USA) 1.5-Tesla whole-body systems (other protocols might be necessary for different systems):

| Protocol SSFSE-T2 MRI | Protocol FAST STIR MRI | Protocol DYNAMIC MRI |
| --- | --- | --- |
| - FOV: 450 mm. | - FOV: 400 mm. | - FOV: 400 mm. |
| - RFOV: 80%. | - RFOV: 75%. | - RFOV: 90%. |
| - TR/TE: Minimum/100 ms. | - TR/TE: 1800/30 ms. | - TR/TE: In phase. |
| | - TI: 165 ms. | - FLIP ANGLE: 10 ms. |
| - THICKNESS/GAP: 8/1 mm. | - THICKNESS/GAP: 7/1mm. | - THICKNESS: 5.0 mm. |
| - NSA: 2. | - NSA: 2. | - NSA: 1. |
| - MATRIX: 320/129. | - MATRIX: 320/320. | - MATRIX: 128/256. |
| - SLICES: 24. | - SLICES: 24. | - SLICES: 128. |
| - ACQUISITION TIME: 40s. | - ACQUISITION TIME: 7:27 min. | - ACQUISITION TIME:17.5 s. |
| | | - PHASES: BASAL |

## Detailed description of the invention

In this invention, the authors of the present invention demonstrate that computerized optical analysis of conventional non-contrast-enhanced MR (magnetic resonance) images of the liver enables the detection of steatohepatitis by NASH-MRI and significant fibrosis by Fibro-MRI in patients suffering from NAFLD. By using the methods of the present invention it is possible to forecast the rate of disease progression, to support therapeutic decision-making, and to monitor potential therapeutic effects.

Diagnoses of liver diseases have long relied on liver biopsy, despite high intra-and inter-observer variability and sampling error. Panel of serum biomarkers to confirm, or rule out, steatohepatitis remained elusive. NASH-test, included in Fibromax®, is a semi-quantitative score with a wide gray zone; Owl-liver® accurately predicts steatohepatitis but needs to be analyzed in a centralized laboratory that undermines accessibility; combination of CK-18 and FGF-21 failed to confirm usefulness in the diagnosis of NASH.

Non-invasive diagnosis of significant fibrosis in NAFLD is also a challenge. NAFLD Fibrosis Score was specifically developed in NAFLD but in the validation process, it showed a wide gray zone with no predictive capacity. Non-invasive markers imported from hepatitis C have been tested in NAFLD like Sydney Index, FIB-4, Forns's Index and APRI. However, a poor correlation between serum biomarkers of liver fibrosis (APRI, FIB4, AST/ALT ratio, European Liver Panel and Liver stiffness measurement) was reported in diabetic patients. Agreement was good on the absence of advanced liver disease but poor agreement on the presence of advanced disease.

Moreover, image-based non-invasive methods are growing interest. Ultrasonography, transient elastography, acoustic radiation force impulse, magnetic resonance spectroscopy, or magnetic resonance elastography have been utilized for NASH diagnosis. Ultrasonography has shown 60-94% sensitivity and 84-95% specificity in hepatic steatosis detection,which is acceptable for first-line steatosis screening in clinical practice, but cannot distinguish NASH from simple steatosis. Transient elastography (FibroScan®; Echosens, Paris, France) has shown a good diagnostic accuracy stratifying advanced fibrosis in NAFLD. However, transient elastography has been found non-useful for the diagnosis of NASH. Nevertheless, more than 10% of patients cannot be assessed because of procedure failures due, mainly, to high body mass index (BMI). Hence, threshold defining advanced fibrosis stage remains controversial and requires higher scores of kPa to define cirrhosis, compared with cut-offs accepted for viral hepatitis. Magnetic resonance spectroscopy (MRS) enables the evaluation, in vivo, of liver molecular composition and the detection of steatosis with high accuracy, being a reference method for steatosis but fails detecting NASH. Magnetic resonance elastography (MRE) has been shown to be accurate in fibrosis staging but its availability is low in most Centers and needs further external validation. The main limitation of these image-based methods has been the inability to detect steatohepatitis. Novel developments in the MR field such as gadolinium probes targeted to type-1 collagen, have shown good preliminary results, but they need to be translated into a standard clinical setting.

In contrast, as shown herein, NASHMRI and FibroMRI can accurately predict steatohepatitis and fibrosis staging. MRI can access deep tissue, and enables whole liver analysis. It is useful in decision-making with respect to donor liver management prior to liver transplantation, or in calculating the speed of fibrosis progression in liver diseases which share steatosis as a major feature such as, for example, viral hepatitis or alcohol-related liver diseases. Since ionizing radiation is avoided, the proposed system is suitable for monitoring fibrosis and steatohepatitis progression over time.

Further, NASHMRI and FibroMRI are independent of the MR system manufacturer (Philips vs. GE, in our current study). Score data are related to the presence of steatohepatitis or fibrosis; the lower the scores the lower the probability of suffering from steatohepatitis, or significant fibrosis. The opposite is also valid i.e. the higher the score the greater the risk of showing steatohepatitis, or significant fibrosis.

Technical limitation is segmentation error. This is because the method is based on an optical analysis of image features to quantify fairly delicate differences that are not visible to the naked eye. Vessels or other structures in the sample could be confounding factors resulting in under- or over-estimating the degree of fibrosis or inflammation. This has been resolved (in the current analysis) by excluding areas containing blood vessels, biliary tract, or focal lesions and, as well, all the samples with >30% pixels outside the segmented area.

In conclusion, NASHMRI and FibroMRI are useful in diagnosing steatohepatitis and significant fibrosis in patients with NAFLD. The methods offer clear advantages over liver biopsy since they are innocuous; enable the screening of large numbers of patients, and the analysis of the whole liver using user-friendly software. The disease could be staged with respect to severity, and could provide information on prognosis, supporting clinical decisionmaking, and monitoring response to therapy.

The following examples merely serve to illustrate the invention and thus they do not limit the same.

## Examples

### MATERIAL AND METHODS

#### - Study design and patients

This was a prospective, multi-centered cohort study that included well-characterized biopsy-proven NAFLD patients. All patients provided informed consent for liver biopsy and MRI study. Inclusion criteria were:

   a. adults (>18 years of age) who showed diffuse hyperechogenic liver on ultrasonography;
   b. impairment in biochemical liver profile;
   c. metabolic syndrome features including an increased HOMA-IR.

Exclusion criteria were significant alcohol abuse (>30 g/day in men and >20 g/day in women) and evidence of viral or autoimmune hepatitis, HIV, drug-induced fatty liver or other metabolic liver diseases (such as hemochromatosis or Wilson's disease), together with pregnancy and parenteral nutrition.

Patients enrolled in the study were classified according to sex, age, fibrosis stage and steatohepatitis presence. Patients underwent a complete medical history, physical examination, blood tests, liver biopsy and imaging study. An overnight (12h) fasting blood sample was taken for routine analyses that included ALT, AST, alkaline phosphatase, $\gamma$GT, cholesterol and triglycerides. Fasting samples of serum obtained after centrifugation were stored in aliquots at -80°C until assayed. Serum insulin levels were measured by electrochemiluminescence immunoassay, using an Elecsys 1010/2010 autoanalyzer (Elecsys MODULAR ANALYTICS E170; Roche, Basil, Switzerland). CK-18 were measured using a human ELISA Kit (Abnova, Walnut, CA, USA). Transient elastography was measured using Fibroscan®, Echosens, France. Height and weight were determined at baseline, and the body mass index (BMI) was calculated as weight (in kg) ÷ height (in m2).

#### - Histological staging and grading

Percutaneous liver biopsies were performed under local anesthesia and ultrasound guidance. Liver specimens were obtained, after an overnight fast, by "tru-cut" needle (sample length/diameter = 20/1.2 mm) using a biopsy gun and, at least, one sample per patient was extracted. Length of liver specimens was recorded and, in conjunction with the number of portal tracts, the sample was considered useful, or not, for histological diagnosis and fibrosis staging. Samples showing <10 mm or <10 portal tracts were excluded.

Biopsies were routinely processed, formalin-fixed and paraffin-embedded. A single pathologist, who was blinded with respect to provenance of the samples, assessed hematoxylin-eosin, reticulin and Masson's trichrome-stains to determine the grading and staging assignments. This scoring system comprises four semi-quantitatively features: steatosis, lobular inflammation, hepatocellular ballooning, and fibrosis. Steatohepatitis presence was calculated according to the Kleiner NAFLD Activity Score (NAS Score) and an additional score for fibrosis. NAS Score provides a composite score that comprises the degree of steatosis (score 0-3), lobular inflammation (score 0-3) and hepatocyte ballooning (score 0-2).

NAS score >5 suggests NASH, while NAS score <3 indicates simple steatosis.

Hepatic steatosis was quantified as the percentage of hepatocytes containing fat droplets, graded on a scale of 0-3 through subjective visual estimation of cells containing fat vacuoles.

Steatosis grades were broadly categorized for severity: grade 0 or normal (up to 5% of hepatocytes affected), grade 1 or mild (5-33% of cells affected), grade 2 or moderate (33- 66% showing steatosis), and grade 3 or severe (>66% of hepatocytes showed fat storage).

Lobular inflammation was assessed as grade 0 (non-inflammation), grade 1 (<2 foci/x200 field), grade 2 (2-4 foci/x200 field) and grade 3 (>4 foci/x200 field).

Ballooning was evaluated as stage 0 (none), stage 1 (a few balloon cells) and stage 2 (many cells or prominent ballooning).

Finally, fibrosis staging was performed according to a 5-level scale: F0=absence; F1=perisinusoidal or periportal; F2=perisinusoidal and portal/periportal, F3=bridging fibrosis, F4=cirrhosis. A further 2-level scale of fibrosis was applied: mild (F0-F1) and advanced fibrosis (F2-F3-F4).

## - Magnetic Resonance Image (MRI) acquisition

MRIs were acquired at the six University Hospitals using General Electric (Milwaukee, CT. USA) or Philips (Best, NL.) 1.5-Tesla whole-body systems. Patients were examined while they were on supine position using a standard torso coil centered over the liver without contrast, heeded by the technologist to individual breath-hold capacity. MRIs were sent to the referral Center for processing. The entire liver was imaged and 6 sections selected. Selected protocols for this study were performed in axial plane, SSFSE-T2 (Single Shot Fast Spin Echo T2-weighted), FAST-STIR (Fast Short inversion Time Inversion Recovery), inPHASE-outPHASE (in and out Phase) and DYNAMIC (See Table 1, Imaging Parameters, below).

| INSTRUCTIONS | - Arms up if possible |
| | - Breath hold must be consistent |
| | - Supine position |
| | - Contrast: none |

| Imaging parameters | Sequence name | | | |
|---|---|---|---|---|
| | SSFSE-T2 | FAST-STIR | In and out of PHASE | DYNAMIC |
| Scan plane | Axial | Axial | Axial | Axial |
| Imaging options | Breath hold | Breath hold | Breath hold | Breath hold |
| Time (sec) | 29.76+10.05 | 340.98 $\pm$ 104.10 | 38.10 $\pm$ 6.66 | 35.44 $\pm$ 90.33 |
| FOV (mm) | 450 | 400 | 410 | 375 |
| Matrix size (px) | 512x512 | 448x448 | 432x432 | 192x192 |
| Sec: seconds; FoV: Field of View; mm: millimeters; px: pixel; | | | | |

DICOM files, field of view (FoV) and matrix sizes were configured specifically for each MR protocol; minimum and maximum window values were calculated, in order to each slice could be converted into a numerical matrix of pixels within that window range. MR Imaging Processing for the definition of NASH-MRi and Fibro-MRi imaging biomarkers

**- Development or selection of imagining biomarkers**

Selected patients according to steatohepatitis and fibrosis stage were included. A squared grid segmented MR images. Sample size ranged from 10x10 to 23x23 pixels, depending on image resolution, to resemble optimal liver biopsy area. All regions containing artifacts, such as vessels, hepatobiliary ducts or with >30% of the pixels outside the segmented area, were discarded and only squares comprising liver parenchyma were analyzed. Both, Philips and G&E MRI were processed.

MRI features, extraction procedures, and implementation were developed in MATLAB® (Matrix Laboratory, MathWorks, Natick, MA, USA) programming language. The software tool developed imports DICOM files and parses them, extracting all needed information such as patient demographic data, denomination and number of MRI assessments performed, number of slices, and date of the images.

a. The whole set of slices corresponding to a new evaluation is shown in a squared grid. Up to 6 consecutive slices must be chosen, preferably those that show the biggest liver section.

b. Selected slices are sequentially shown separately. The user must draw the outline of liver boundaries to segment the whole liver. Once segmented, a grid of squares is automatically over-layered on the segmented area. To achieve a sample size equivalent to 20 mm of tissue, the number of pixels of samples is computed using FoV value and number of rows and columns of the image matrix. Therefore, the numbers of samples vary for different MRI sequences.

c. The software automatically discards those samples with >30% of the surface outside the segmentation line. The user must also discard samples that do not represent homogeneous liver tissue.

d. A total of 84 different mathematical parameters are computed from each sample. The nature of these parameters ranges from simple statistical descriptors such as mean and standard deviation, to energy features like entropy, geometrical properties such as mean surface curvature, and spectrum characteristics.

e. These big data was processed using architectural neural networks and logistic regression. The combination of parameters from the different protocols able to define two imaging biomarkers to predict NASH (NASH-MRi) and fibrosis (Fibro-MRi) were selected.

**- Validation of imaging biomarkers**

Developed imaging biomarkers were validated. No differences were seen according age, gender, steatohepatitis or fibrosis distribution. NASH was present in 44 cases (51%), and significant fibrosis in 33 patients (38%). Fibrosis distribution among the patients was: F0, n=39, F1, n=17, F2, n=18, F3, n=9, F4: n=4. Three protocols were required to accurately detect steatohepatitis and two for fibrosis detection. The average time consumed was 11.23+3.4 minutes.

**- Comparison with biochemical biomarkers**

NASH-MRi was compared with CK-18 and FGF-21 levels and Fibro-MRi with FIB-4, Sydney Index and NAFLD Fibrosis Score using under area ROC curves (AUROC).

**- Data analysis and statistics**

Software package SPSS® 22.0 (SPSS, Chicago, IL, USA) was used to record data and to perform detailed statistical analysis. Values of $p < 0.05$ were considered statistically significant. Multiple linear regression analysis was used to compare continuous variables.

Receiver operating characteristics (ROC) curves, which represent the tradeoff between the true and false positive rate, were used to differentiate the misclassified data between normal and disease status. The AUROC can vary from 0.5 (random discriminatory accuracy) to 1.0 (perfect discriminatory accuracy)

AUROC was generated in order to evaluate the diagnostic accuracy of the tools as well as the predictive proficiency with respect to the presence of mild fibrosis (F0-F1), significant fibrosis (F2-F3-F4), and steatohepatitis. NASHMRI and FibroMRI were the outputs of the optical analysis and were defined as predictive models used to foretell steatohepatitis and significant fibrosis. Concordance between numerically calculated NASHMRI and FibroMRI values and histopathologically defined steatohepatitis and fibrosis were compared by Spearman regression coefficient analysis. Sample size was calculated to show a significant difference between histological parameters and NASHMRI and FibroMRI using nQuery advisor v7.0 software.

Table 2. Baseline characteristics of patient population: demography, laboratory and histology results. Values presented as mean ± SD, unless otherwise stated

| Parameter | Overall cohort (N= 126) |
|---|---|
| Age; y | 51±12 |
| Male gender; % | 66 |
| Body mass index; kg/m$^2$ | 30.59±4.84 |
| Waist circumference; cm | 101.8±11.11 |
| Hispanic ethnicity; % | 100 |
| Arterial hypertension; % | 36.4 |
| Diabete s; % | 37.5 |
| Cholesterol; mmol/L | 8.53±10.9 |
| Triglycerides; mmol/L | 5.87±11.71 |
| ALT; IU/L | 73±44 |
| AST; IU/L | 46±39 |
| GGT; IU/L | 101±101 |
| Platelet count; x10$^9$ | 233±57 |
| Fasting glucose; mmol/L | 8.06±15.72 |
| Insulin; mg/dL | 14.88±9.29 |
| Steatosis grade; % | |
| 0 | 17.9 |
| 1 | 42.3 |
| 2 | 24.4 |
| 3 | 15.4 |
| NASH; % | 51.2 |
| Fibrosis stage; % | |
| F0-F1 | 60 |
| F2-F3-F4 | 40 |
| NAS Score (%) | |
| <4 | 37.1 |
| ≥4 | 62.9 |

Notes to Table 2: AST: Aspartate Aminotransferase; ALT:Alanine aminotransferase; GGT: Gamma Glutamyl Transferase; NASH: Non-alcoholic Steatohepatitis; NAS score: NAFLD Activity Score.

**Example 1. Development and standardization of NASH-MRI for detection of steatohepatitis**

Estimator E3 (harmonic mean) from MRI protocol SSFSE-T2, estimator E57 (2-2 order contrast) from MRI protocol DYNAMIC, and estimator E73 (weighted mean curvature) from MRI protocol FAST-STIR, were found to be independently associated with NASH.

Model coefficients associated with each one of these independent variables were β1 =0.079 (OR: 1.08, 95%CI: 1.02-1.15; p=0.015) and β2=0.22 (OR: 1.14, 95%CI: 1.03-1.26; p=0.015).

These estimators influence on the predictive equation to obtain the probability of suffering steatohepatitis:

$$\text{NASH-MRI} = 1/\left(1 + e^{(1.654 - 0.079*E3\_T2 - 0.127*E57\_DYN*E73\_FAST)}\right)$$

AUROC obtained was 0.88 (95%CI: 0.77-0.99) in the estimation cohort. Values for sensitivity of 87%, specificity of 74%, positive predictive value (PPV) of 80% and negative predictive value (NPV) of 82% were obtained from prediction based on a cut-off point of 0.5 for NASHMRI.

In the validation cohort, NASH-MRI AUROC was 0.84 (95%CI: 0.75-0.92). Using the defined threshold of 0.5 for NASH-MRI prediction, results obtained were: sensitivity 87%, specificity 60%, PPV 71% and NPV 81%.

**Example 2. Development and standardization of Fibro-MRI for detection of fibrosis**

Estimator E22 (Pearson's asymmetry coefficient) from MRI protocol SSFSE-T2 and estimators E3 (harmonic mean), E6 (mode), E31 (column's mean of multi-oriented co-occurrence matrix) and E75 (maximum of main curvatures) from MRI protocol DYNAMIC were found to be independently associated with fibrosis. Model coefficients associated with each of these independent variables were: $\beta1=1.101$ (OR: 3.01, 95%CI: 1.25-7.25; p=0.014); $\beta2=-1.105$ (OR: 0.33, 95%CI: 0.14-0.77; p=0.010); $\beta3=-115.737$ (OR: 0.08, 95%CI: 0.02-0.14; p=0.046) $\beta4=0.696$ (OR: 2.00, 95%CI:1.19-3.38; p=0.009); and $\beta5=-0.825$ (OR: 0.44,v95CI%: 0.21-0.93; p=0.030) should be introduced into the predictive equation to obtain the risk of suffering fibrosis as:

$$\text{Fibro-MRI} = 1/\left(1+e^{(-4.207-1.101*E3\_DYN + 1.105*E6\_DYN + 115.737*E22\_T2 - 0.696*E31\_DYN + 0.825*E75\_DYN)}\right)$$

In the estimation cohort, AUROC was 0.94 (95%CI: 0.87-1.00) with a sensitivity of 81%, specificity of 85%, PPV of 77% and NPV of 86% using a cut-off point of 0.5 for FibroMRI.

In the validation cohort, Fibro-MRI AUROC for significant fibrosis was 0.85 (95%CI: 0.77-0.93). Using the defined threshold at 0.5 for FibroMRI prediction, the results obtained were: sensitivity 77%, specificity 80%, PPV 67% and NPV 87%.

**Example 3. Standardization of NASH-MRI and Fibro-MRI across MRI systems**

NASHMRI, calculated using a GE scanner, showed a similar diagnostic accuracy AUROC=0.76 (95%CI: 0.58-0.96) vs. AUROC=0.83 (95%CI: 0.70-0.96); p=ns in comparison with NASHMRI calculated in patients that underwent MRI with the Philips system. With respect to FibroMRI, evaluations performed using a GE scanner showed an AU-ROC=0.81 (95%CI: 0.66-0.95) vs. AUROC=0.86 (95%CI: 0.72-0.99) using the Philips system (p=ns).

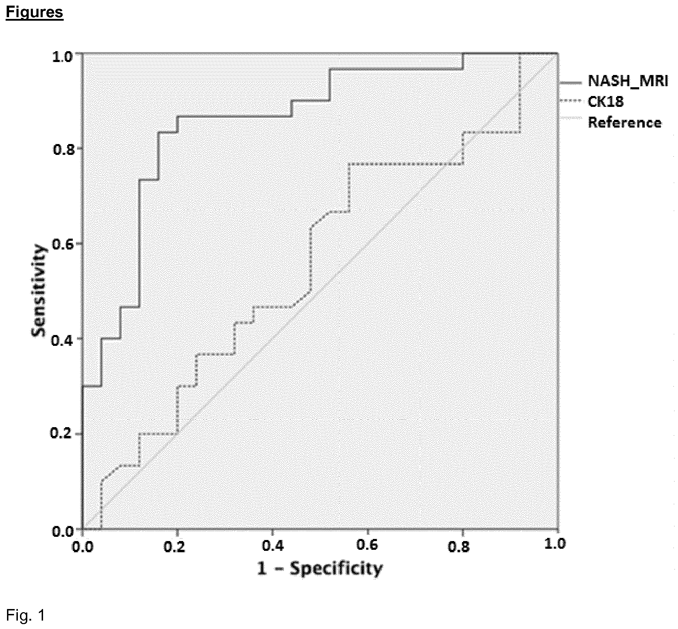

Comparative analysis with non-invasive biochemical markers of steatohepatitis NASHMRI was compared with FGF-21 and CK-18 in the diagnosis of steatohepatitis in a cohort of 64 patients. NASHMRI offered the best diagnostic accuracy with an AUROC of 0.86 (95%CI: 0.76-0.96) for steatohepatitis presence significantly better than CK-18 levels AUROC of 0.56 (95%CI: 0.40-0.71; p<0.05) (Figure 1). NAS score correlated with both NASH-MRI (r=0.38;p<0.001) and CK-18 levels (r=0.29;p<0.02).

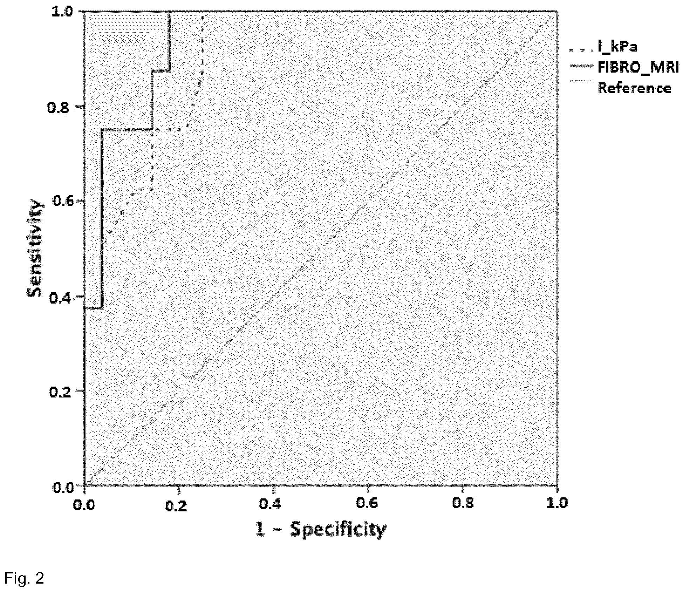

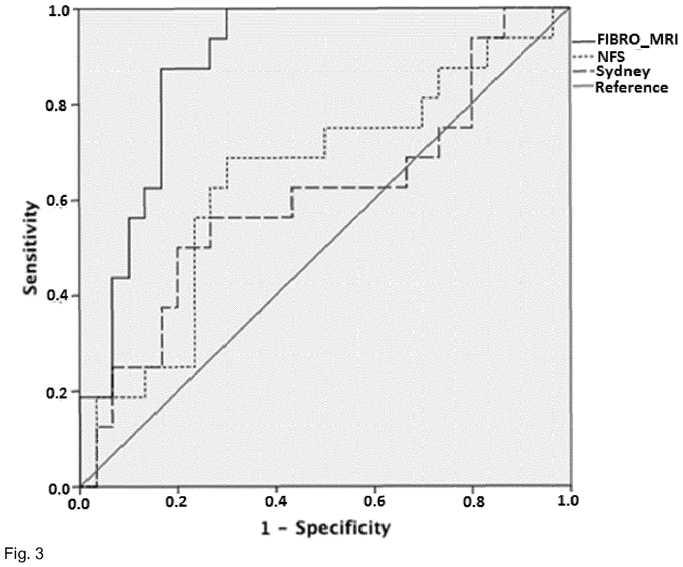

Comparative analysis with non-invasive biochemical markers of significant fibrosis Fibro-MRI (AUROC: 0.85; 95%CI: 0.74-0.97) was significantly superior to NFS (AUROC: 0.56; 95%CI:0.41-0.71) and Sydney Index (AUROC: 0.69; 95%CI:0.50-0.87); p<0.05 (Figure 2). Fibrosis stage correlated with Fibro-MRI (r=0.61;p<0.001), and with NFS (0.52;p<0.001). Moreover, a correlation between NFS and Fibro-MRI was found (r=0.53;p<0.001). Lastly, performance of FibroMRI (AUROC: 0.95; 95%CI: 0.88-1.00) was similar to transient elastography (AUROC: 0.91;95%CI: 0.81-1.00);p=ns (Figure 3).

**Claims**

1. An non-invasive method for quantifying the lesions of the liver of a subject suspected of suffering from a non-alcoholic fatty liver disease addressing as diagnostic target steatohepatitis, which comprises:

   a. performing a T2-weighted single shot fast spin echo magnetic resonance imaging (SSFSE-T2 MRI) and a Dynamic contrast-enhanced magnetic resonance imaging (DYNAMIC MRI) and a fast spin-echo short inversion time inversion-recovery (STIR MRI) magnetic resonance imaging (FAST-STIR MRI), on whole or part of the liver of the subject,
   b. measuring a harmonic mean (E3) from the whole set of pixel values of an image generated with said SSFSE-T2 MRI, an order contrast (E57) from an image generated with said DYNAMIC MRI and the averaged main curvature (E73) from an image generated with said FAST-STIR MRI; and
   c. using said measurements in a mathematical function useful as a predictive equation to obtain the probability of suffering from steatohepatitis;
   wherein the mathematical function useful as a predictive equation is as follows:

   $$\text{NASH-MRI} = 1/\left(1 + e^{1.654 - 0.079*E3\_T2 - 0.127*E57\_DYN*E73\_FAST}\right)$$

   wherein:

E3_T2 = harmonic mean (E3) from the image generated with the T2-weighted single shot fast spin-echo magnetic resonance imaging (SSFSE-T2 MRI).
E57_DYN = order contrast (E57) from the image generated with the dynamic contrast-enhanced magnetic resonance imaging (DYNAMIC MRI)
E73_FAST = averaged mean curvature (E73) from the image generated with the fast spin-echo short inversion time inversion-recovery (FAST-STIR MRI);

and wherein the MRIs images are each acquired by using a General Electric 1,5-Tesla whole-body system.

**2.** An non-invasive method for quantifying the lesions of the liver of a subject suspected of suffering from a non-alcoholic fatty liver disease addressing as diagnostic target fibrosis, which comprises:

a. performing a T2-weighted single shot fast spin echo magnetic resonance imaging (SSFSE-T2 MRI) and a Dynamic contrast-enhanced magnetic resonance imaging (DYNAMIC MRI), on whole or part of the liver of the subject,
b. measuring the Pearson's asymmetry coefficient (E22) from an image generated with the SSFSE-T2 MRI and measuring a harmonic mean (E3), a mode (E6), a column's mean of a co-ocurrence matrix (E31) and the maximum of main curvatures (E75) from an image generated with the DYNAMIC MRI; and
c. using said measurements in a mathematical function useful as a predictive equation to obtain the probability of suffering from fibrosis
wherein the mathematical function useful as a predictive equation is as follows:

$$\text{Fibro-MRI} = 1/(1+e^{-4.207-1.101*E3\_DYN + 1.105*E6\_DYN + 115.737*E22\_T2 - 0.696*E31\_DYN + 0.825*E75\_DYN})$$

wherein:

E3_DYN = harmonic mean (E3) from the dynamic contrast-enhanced magnetic resonance imaging (DYNAMIC MRI).
E6_DYN = mode (E6) from the dynamic contrast-enhanced magnetic resonance imaging (DYNAMIC MRI).
E22_T2 = Pearson's asymmetry coefficient (E22) from the T2-weighted single shot fast spin-echo magnetic resonance imaging (SSFSE-T2 MRI).
E31_DYN = Column's mean of multi-oriented co-occurrence matrix (E31) from the dynamic contrast-enhanced magnetic resonance imaging (DYNAMIC MRI).
E75_DYN = Maximum of main curvatures (E75) from the dynamic contrast-enhanced magnetic resonance imaging (DYNAMIC MRI);

and wherein the MRIs images are each acquired by using a General Electric 1,5-Tesla whole-body system.

**3.** A computerized optical analysis method of the images of the liver obtained in step a) of any of claims 1-2, which comprises using means configured to perform steps b) and c) of any of claims 1-2 in order to obtain the probability of suffering from the diagnostic target.

**4.** A computer program capable of implementing the computerized optical analysis method of claim 3.

**5.** A device comprising the computer program of claim 4.

**Patentansprüche**

**1.** Nichtinvasives Verfahren zur Quantifizierung von den Leberläsionen einer Testperson, von der es vermutet wird, dass sie unter einer nichtalkoholischen Fettlebererkrankung leidet, unter Auswahl der Steatohepatitis als Diagnoseziel, welches Folgendes umfasst:

a. das Durchführen einer T2-gewichteten Single-Shot-Fast-Spin-Echo-Magnetresonanzbildgebung (SSFSE-T2 MRI) und einer dynamischen kontrastverbesserten Magnetresonanzbildgebung (DYNAMIC MRI) und einer Fast-Spin-Echo-Short-Time Inversion Recovery (STIR MRI)-Magnetresonanzbildgebung (FAST-STIR MRI), auf der gesamten Leber der Testperson oder auf einem Teil derselben,

b. das Messen eines harmonischen Mittels (E3) aus dem Gesamtsatzes von Pixelwerten eines mit der genannten SSFSE-T2 MRI erzeugten Bildes, eines Ordnungskontrasts (E57) eines mit der genannten DYNAMIC MRI erzeugten Bildes und der gewichteten Hauptkrümmung (E73) eines mit der genannten FAST-STIR MRI erzeugten Bildes; und

c. das Verwenden der genannten Messungen in einer mathematischen Funktion, welche als Vorhersagegleichung nützlich ist, um die Wahrscheinlichkeit des Leidens unter Steatohepatitis zu erhalten; wobei die mathematische Funktion, welche als Vorhersagegleichung nützlich ist, wie folgt ist:

$$NASH\text{-}MRI = 1/ (1 + e^{1,654 - 0,079*E3\_T2 - 0,127*E57\_DYN*E73\_FAST})$$

in welcher:

E3_T2 = harmonisches Mittel (E3) des mit der T2-gewichteten Single-Shot-Fast-Spin-Echo-Magnetresonanzbildgebung (SSFSE-T2 MRI) erzeugten Bildes;
E57_DYN = Ordnungskontrast (E57) des mit der dynamischen kontrastverbesserten Magnetresonanzbildgebung (DYNAMIC MRI) erzeugten Bildes;
E73_FAST = gewichtete mittlere Krümmung (E73) des mit der Fast-Spin-Echo-Short-Time Inversion Recovery (FAST-STIR MRI) erzeugten Bildes;

und wobei die MRI-Bilder jeweils unter Verwendung eines General Electric 1,5-Tesla Ganzkörpersystems erworben werden.

2. Nichtinvasives Verfahren zur Quantifizierung von den Leberläsionen einer Testperson, von der es vermutet wird, dass sie unter einer nichtalkoholischen Fettlebererkrankung leidet, unter Auswahl der Fibrose als Diagnoseziel, welches Folgendes umfasst:

a. das Durchführen einer T2-gewichteten Single-Shot-Fast-Spin-Echo-Magnetresonanzbildgebung (SSFSE-T2 MRI) und einer dynamischen kontrastverbesserten Magnetresonanzbildgebung (DYNAMIC MRI), auf der gesamten Leber der Testperson oder auf einem Teil derselben,
b. das Messen des Pearson-Asymmetriekoeffizienten (E22) eines mit der SSFSE-T2 MRI erzeugten Bildes und das Messen eines harmonischen Mittels (E3), eines Modus (E6), eines Spaltenmittels einer Grauwertematrix (E31) und des Maximums von Hauptkrümmungen (E75) eines mit der DYNAMIC MRI erzeugten Bildes; und
c. das Verwenden der genannten Messungen in einer mathematischen Funktion, welche als Vorhersagegleichung nützlich ist, um die Wahrscheinlichkeit des Leidens unter Fibrose zu erhalten
wobei die mathematische Funktion, welche als Vorhersagegleichung nützlich ist, wie folgt ist:

$$Fibro\text{-}MRI= 1/ (1+e^{-4,207-1,101*E3\_DYN + 1,105*E6\_DYN + 115,737*E22\_T2 - 0,696*E31\_DYN + 0,825*E75\_DYN})$$

in welcher:

E3_DYN = harmonisches Mittel (E3) der dynamischen kontrastverbesserten Magnetresonanzbildgebung (DYNAMIC MRI);
E6_DYN = Modus (E6) der dynamischen kontrastverbesserten Magnetresonanzbildgebung (DYNAMIC MRI);
E22_T2 = Pearson-Asymmetriekoeffizient (E22) der T2-gewichteten Single-Shot-Fast-Spin-Echo-Magnetresonanzbildgebung (SSFSE-T2 MRI);
E31_DYN = Spaltenmittel der multi-orientierten Grauwertematrix (E31) der dynamischen kontrastverbesserten Magnetresonanzbildgebung (DYNAMIC MRI);
E75_DYN = Maximum der Hauptkrümmungen (E75) der dynamischen kontrastverbesserten Magnetresonanzbildgebung (DYNAMIC MRI);

und wobei die MRI-Bilder jeweils unter Verwendung eines General Electric 1,5-Tesla Ganzkörpersystems erworben werden.

3. Rechnergestütztes optisches Analyseverfahren von den Bildern der Leber erhalten in Schritt a) nach einem der Ansprüche 1-2, welches das Verwenden von Mitteln umfasst, welche dazu ausgebildet sind, Schritte b) und c) nach einem der Ansprüche 1-2 durchzuführen, um die Wahrscheinlichkeit des Leidens unter dem Diagnoseziel zu erhalten.

4. Computerprogramm, welches in der Lage ist, das rechnergestützte optische Analyseverfahren nach Anspruch 3 zu realisieren.

5. Vorrichtung umfassend das Computerprogramm nach Anspruch 4.

**Revendications**

1. Procédé non invasif pour la quantification de lésions du foie d'un sujet suspecté de souffrir d'une stéatose hépatique non alcoolique remédiant à une stéatohépatite de cible diagnostique, qui comprend :

   a. mettre en oeuvre une imagerie par résonance magnétique de écho de spin rapide d'impulsion d'inversion unique pondéré en T2 (IRM SSFSE-T2) et une imagerie par résonance magnétique dynamique avec produit de contraste (IRM dynamique) et une imagerie par résonance magnétique (IRM FAST-STIR) d'inversion récupération de temps de écho de spin rapide d'inversion courte (IRM STIR), sur tout ou une partie du foie du sujet,
   b. mesurer une moyenne harmonique (E3) de tout l'ensemble de valeurs de pixels d'une image générée par ladite IRM SSFSE-T2, un contraste de commande (E57) d'une image générée par ladite IRM dynamique et la courbure principale moyenne (E73) d'une image générée par ladite IRM FAST-STIR ; et
   c. utiliser lesdites mesures dans une fonction mathématique utile comme équation prédictive pour obtenir la probabilité de souffrir d'une stéatohépatite :

   dans lequel la fonction mathématique utile comme équation prédictive est la suivante :

$$\text{IRM pour NASH} = 1/ \left(1+ e^{1,654-0,079*E3\_T2-0,127*E57\_DYN*E73\_FAST}\right)$$

   dans laquelle:

   E3_T2 = moyenne harmonique (E3) de l'image générée par l'imagerie par résonance magnétique de écho de spin rapide d'impulsion d'inversion unique pondéré en T2 (IRM SSFSE-T2),
   E57_DYN = contraste de commande (E57) de l'image générée par l'imagerie par résonance magnétique dynamique avec produit de contraste (IRM dynamique)
   E73_FAST = courbure moyenne (E73) de l'image générée par d'inversion récupération de temps de écho de spin rapide d'inversion courte (IRM FAST-STIR) ;

   et dans lequel les images IRM, sont chacune acquises en utilisant un système de corps entier General Electric 1,5-Tesla.

2. Procédé non invasif pour la quantification de lésions du foie d'un sujet suspecté de souffrir d'une stéatose hépatique non alcoolique remédiant à une fibrose de cible diagnostique, qui comprend :

   a. mettre en oeuvre une imagerie par résonance magnétique de écho de spin rapide d'impulsion d'inversion unique pondéré en T2 (IRM SSFSE-T2) et une imagerie par résonance magnétique dynamique avec produit de contraste (IRM dynamique) sur tout ou une partie du foie du sujet,
   b. mesurer le coefficient d'asymétrie de Pearson (E22) d'une image générée par IRM SSFSE-T2 et mesurer une moyenne harmonique (E3), un mode (E6), une moyenne de colonne d'une matrice de concomitance (E31) et le maximum des courbures principales (E75) d'une image générée par IRM dynamique; et
   c. utiliser lesdites mesures dans une fonction mathématique utile comme équation prédictive pour obtenir la probabilité de souffrir de fibrose
   dans lequel la fonction mathématique utile comme équation prédictive est la suivante :

$$\text{IRM pour fibrose} = 1/ \left(1+ e^{-4,207-1,101*E3\_DYN+1,105*E6\_DYN+115,737*E22\_T2-0,0696*E31\_DYN+0,825*E75\_DYN}\right)$$

   dans laquelle:

E3_DYN = moyenne harmonique (E3) de l'image générée par l'imagerie par résonance magnétique dynamique avec produit de contraste (IRM dynamique),

E6_DYN = mode (E6) d'imagerie par résonance magnétique dynamique avec produit de contraste (IRM dynamique)

E22_T2 = coefficient d'asymétrie de Pearson (E22) de l'imagerie par résonance magnétique de écho de spin rapide d'impulsion d'inversion unique pondéré en T2 (IRM SSFSE-T2),

E31_DYN = moyenne de colonne d'une matrice de concomitance multi-orientée (E31) de l'imagerie par résonance magnétique dynamique avec produit de contraste (DYNAMC IRM),

E75_DYN = Maximum de courbures principales (E75) de l'imagerie de résonance magnétique dynamique avec produit de contraste (IRM dynamique)

et dans lequel les images IRM sont chacune acquises en utilisant un système de corps entier General Electric 1,5-Tesla.

3. Procédé d'analyse optique informatisée d'images du foie obtenues dans l'étape a) selon l'une quelconque des revendications 1-2, qui comprend en utilisant des moyens configurés pour mettre en oeuvre les étapes b) et c) selon l'une quelconque des revendications 1-2 afin d'obtenir la probabilité de souffrir de la cible diagnostique.

4. Programme informatique apte à mettre en oeuvre le procédé d'analyse optique informatisée selon la revendication 3.

5. Dispositif comprenant le programme informatique selon la revendication 4.

**Figures**

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005116901 A

- WO 2006082522 A

### Non-patent literature cited in the description

- **HUERTAS, R. ; GEBEJES, A.** Texture Characterization based on Grey-Level Co-occurrence Matrix. *Conference of Informatics and Management Sciences,* 2013, 375-378